# EUROPÄISCHE PATENTSCHRIFT

(11) **EP 1 732 466 B1**
(45) Veröffentlichungstag und Bekanntmachung des Hinweises auf die Patenterteilung: **05.06.2013**
(21) Anmeldenummer: 05752569.3
(22) Anmeldetag: 01.04.2005
(51) Int. Cl.: A61F 2/00

(54) **INSTRUMENTARIUM ZUM FIXIEREN EINES IMPLANTATES IN EINEM KNOCHEN**
INSTRUMENT FOR FIXING AN IMPLANT IN A BONE
INSTRUMENT POUR FIXER UN IMPLANT DANS UN OS

(30) Priorität: 07.04.2004 DE 102004018426
(43) Veröffentlichungstag der Anmeldung: 20.12.2006
(73) Patentinhaber: Karl Storz GmbH & Co. KG, 78532 Tuttlingen (DE)
(72) Erfinder: HIRT, Hubert, 79100 Freiburg (DE); BERBERICH, Sascha, 78532 Tuttlingen (DE)
(74) Vertreter: Weller, Wolfgang
(86) Internationale Anmeldenummer: PCT/EP2005/003431
(87) Internationale Veröffentlichungsnummer: WO 2005/096987

(56) Entgegenhaltungen:
- EP-A- 0 279 129
- EP-A- 0 425 140
- DE-U1- 29 922 088
- US-A- 3 973 277
- US-A1- 2003 065 391
- US-B1- 6 540 783

## Beschreibung

Die vorliegende Erfindung betrifft ein Instrumentarium zum Fixieren eines strangförmigen Implantats in einem Knochen, mit einem im Knochen einsetzbaren Halteelement, wobei das Halteelement eine Ausnehmung zur Aufnahme eines Querbolzens sowie Mittel, um es lösbar mit einem Applikator zu verbinden, aufweist und ferner zur Fixierung des Implantats dient, wobei der Querbolzen das Halteelement in dem Knochen verankert, und wobei das Halteelement aus einem biodegradierbarem Material besteht.

Ein derartiges Instrumentarium ist aus der US 2003/0065391 A1 bekannt.

In der modernen Chirurgie müssen häufig strangförmige Implantate in einem Knochen fixiert werden. So werden z.B. bei der Rekonstruktion der Bänder im Kniegelenk natürliche oder künstliche Ersatzbänder in einen Kanal im Femur eingebracht, und dort mittels eines Querbolzens fixiert.

Es ist auch bekannt, Fadenschleifen durch einen Querbolzen in einem Knochen zu verankern und diese Fadenschleifen z.B. zur Befestigung von abgelösten oder gerissenen Stücken der Rotatorenmanschette am Kopf des Humerus zu verwenden. Die Befestigung kann dabei direkt, also durch die Fadenschleifen selbst, oder durch Mittel erfolgen, die mit den Fadenschleifen verbunden sind.

Werden solche strangförmigen Implantate ohne ein Halteelement, nur durch den Querbolzen in einem Kanal im Knochen fixiert, kann es unter Belastung zu einer Hin- und Herbewegung des Implantates kommen. Diese Hin- und Herbewegung stört erstens das Anwachsens eines solchen Implantates und kann zweitens zu einem Scheuern des Implantates auf dem Querbolzen führen, was im Extremfall zum Reißen des Implantats führt.

Es wurde daher versucht, diese Hin- und Herbewegung mittels eines Halteelements zu vermindern.

Aus der eingangs genannten US 2003/0065391 A1 ist eine Vorrichtung und ein Verfahren zur Rekonstruktion eines Bandes im Knie bekannt. Diese Vorrichtung beinhaltet ein Halteelement, das zumindest zwei durchgehende Öffnungen aufweist. Die erste Öffnung dient zur Aufnahme des strangförmigen Implantats, während die zweite Öffnung zur Aufnahme des Querbolzens dient.

Zur Rekonstruktion eines Bandes gemäß der US 2003/0065391 A1 wird das Implantat durch das proximale Loch hindurchgeführt und das Halteelement mittels eines Applikators in einen im Femur vorbereiteten Kanal eingeführt. Das Halteelement wird dann in einem zweiten Schritt durch einen in das distale Loch des Halteelements eingeführten Querbolzen im Femur verankert.

Auch wenn durch ein solches Halteelement die Hin- und Herbewegung eines Bandes in einem Knochenkanal deutlich vermindert werden kann, weist diese Konstruktion das Problem auf, dass bei starken Zugkräften das Halteelement auseinander gerissen werden kann, so dass sich das Band vom Halteelement löst. Im Körper verbleibt dann der Querbolzen und der Rest des Halteelements.

In diesem Fall müssen diese Bruchstücke entfernt und erneut ein Halteelement gesetzt werden.

Aus der EP 0 425 140 A2 ist eine Vorrichtung zum Verankern eines strangförmigen Implantats in einen Knochen bekannt. Hierbei wird das strangförmige Implantat zuerst mit einer steckerartigen Vorrichtung verbunden und die Kombination aus der steckerartigen Vorrichtung und dem Implantat wird dann mit einer mittels eines Außengewinde in einem Knochen befestigten Buchse verbunden.

Aus der DE 299 22 088 U1 ist eine Vorrichtung zur Befestigung eines strangförmigen Implantats an einem Knochen bekannt. Diese Vorrichtung besteht aus einer ersten Platte, die mit einem hohlen Schaft mit einem Außengewinde und einem Innengewinde verbunden ist und die mittels des Außengewindes des hohlen Schafts mit einem Knochen verbunden werden kann, und aus einer zweite Platte, die mittels einer Schraube, die in das Innengewinde des hohlen Schafts eingreift, gegen die erste Platte verspannbar ist. Das Implantat kann dabei zwischen den beiden Scheiben eingequetscht werden.

Aus der EP 0 279 129 A1 ist eine Vorrichtung zur Befestigung eines Implantats an einem Knochen bekannt. Diese Vorrichtung weist eine Scheibe auf an der ein strangförmiges Implantat befestigt werden kann. Diese Scheibe wird mittels einer exzentrisch darin angeordneten Schraube an einem Knochen befestigt, wobei durch Verdrehen der Scheibe gegenüber der Schraube eine auf das Implantat ausgeübte Spannung verändert werden kann. Durch weiteres Anziehen der Schraube greifen an der Scheibe befestigte Zapfen in den Knochen ein und legen die Position der Scheibe fest.

Aus der US 3,973,277 ist ein Implantat zur Befestigung von strangförmigen Implantaten bekannt, das einen sich verjüngenden porösen Pfropfen vorzugsweise aus gesintertem Metall ausweist.

Aus der US 6,504,783 B1 ist ein Verfahren zum Befestigen eines Knochenblocks in einem Knochentunnel unter Verwendung von zwei Querbolzen bekannt.

Es ist daher Aufgabe der Erfindung, ein Instrumentarium zum Fixieren eines strangförmigen Implantats in einem Knochen so weiterzubilden, dass damit ein strangförmiges Implantat mit deutlich höherer Sicherheit und Zuverlässigkeit in einem Knochen fixiert werden kann.

Erfindungsgemäß wird diese Aufgabe dadurch gelöst, dass das Halteelement eine Führung zur Aufnahme des Implantats aufweist, die so ausgebildet ist, dass das Implantat von dem Halteelement distalseitig schleifenartig um den Querbolzen herumführbar ist.

Kommt es aufgrund starker Zugkräfte zu einem Brechen des Halteelements, führt dieses nicht mehr zu einem Ausreißen des strangförmigen Implantats, da dieses dann noch durch den Querbolzen gegen ein Abziehen gehalten wird.

Da ein Versagen des Haltelements außerdem auf Grund der hohen Belastung meist im Bereich der Krafteinleitungsstellen erfolgt, die bei Zug im Bereich des Scheitels der Schleife liegen, bleiben die Teile der Führung erhalten, die sich seitlich des Implantats befinden, so dass auch bei Bruch eine Hin- und Herbewegung des Implantats zumindest gemindert, wenn nicht gar vollständig vermieden wird.

Die schleifenartige Führung des Implantates distalseitig um das Halteelement herum schafft eine relativ große Anlagefläche zwischen Implantat und Halteelement. Dadurch wird die Gefahr, dass das Implantat an kantenartigen Bereichen scheuert und reißt erheblich vermindert.

Ein erfindungsgemäßes Instrumentarium mit einem solchen Halteelement kann somit, auch über lange Zeiträume, ein Implantat sicher in einem Knochen verankern. Das im Körper verbleibende Halteelement ist in dieser Allgemeinheit das Instrumentarium.

In einer weiteren Ausgestaltung der Erfindung ist die Führung als eine zur Außenseite offene Führung ausgebildet.

Es kann sich hierbei z.B. um eine Nut handeln, in die das Implantat eingelegt wird.

Eine solche Art der Führung hat den Vorteil, dass das Implantat einfach von außen in die offene Führung eingelegt werden kann, wodurch das Einbringen des Implantats in die Führung sehr einfach ist. Dies ist besonders vorteilhaft bei steifen Implantaten, wie z.B. bei Drähten oder auch bei natürlichen Implantaten, die häufig glitschig und schwierig zu handhaben sind.

Diese Maßnahme hat weiter den Vorteil, dass die Führung in der Herstellung einfach ist. Ein Halteelement mit einer offenen Führung kann z.B. durch Spritzgießen gleich in der gewünschten Form gefertigt werden oder die Führung kann z.B. in Form einer Nut in einem Rohling des Halteelements gefräst werden.

In einer weiteren Ausgestaltung der Erfindung, ist die Führung als geschlossene Führung ausgebildet.

Unter einer geschlossenen Führung ist eine Führung zu verstehen, die zumindest auf einem Teil ihrer Länge vollumfänglich geschlossen ist. Die Führung kann hierbei auf ihrer gesamten Länge rohrförmig ausgebildet sein, oder sie kann sich aus einzelnen Rohrabschnitten zusammensetzen. Es kann auch ein Schlitz vorhanden sein, über den das Implantat in die Führung eingeclipst werden kann. Es ist auch möglich, ein Halteelement mit bereits vorgefertigtem Implantat in Form eines Fadens bereitzustellen, wobei der Faden bereits in die geschlossene Führung eingebracht ist.

Da die Führung zumindest auf einem Teil ihrer Länge voll umfänglich geschlossen ist, kann es nicht mehr zu einem Herausfallen oder einem seitlichen Herausrutschen des Implantats aus der Führung und somit einem Ablösen vom Halteelement kommen.

In einer weiteren Ausgestaltung steht eine Mittelachse eines in die Ausnehmung eingeführten Querbolzens senkrecht auf einer durch die Führung definierten Schleifenebene.

Diese Ausgestaltung hat den Vorteil, dass dadurch ein kleinstmögliches Halteelement hergestellt werden kann, da der Querbolzen auf der kürzestmöglichen Strecke schleifenartig umschlungen wird.

In einer weiteren Ausgestaltung ist das Haltelement U-förmig ausgebildet.

Eine U-förmige Ausgestaltung bedeutet, dass ein distaler Abschnitt des Halteelements in etwa halbkreisförmig ausgebildet ist und ein proximaler Abschnitt des Halteelements aus zwei sich von distal nach proximal erstreckenden Armen gebildet wird.

Der halbkreisförmige Abschnitt verjüngt sich in distaler Richtung, was das Einführen des Halteelements in einen Knochen erleichtert.

Die Arme des proximalen Abschnitts verhindern, dass sich das Halteelement während oder nach der Implantation in den Knochen um den Querbolzen verdreht, was zu Belastungen des Implantats bzw. von mit dem Implantat verbundenem Gewebe führen kann.

Die Arme des proximalen Abschnitts können außerdem vorteilhafterweise zum Verbinden des Halteelements mit einem Applikator genutzt werden.

In einer weiteren Ausgestaltung umschlingt die Führung die Ausnehmung um zumindest 180°.

Je größer der Winkel, in dem die Führung die Ausnehmung umschlingt, desto länger die Wegstrecke, auf der das Implantat geführt wird. Je länger die Wegstrecke, auf der das Implantat geführt wird, desto sicherer wird das Implantat um den Querbolzen herumgeführt.

Außerdem vergrößert sich durch ein Verlängern dieser Wegstrecke die Auflagefläche des Implantats auf dem Halteelement. Dadurch wird die Belastung des Halteelements auf eine größere Fläche verteilt, wodurch sich die Haltbarkeit des Halteelements erhöht.

In einer weiteren Ausgestaltung weist ein proximales Ende des Halteelements einen viereckigen Querschnitt auf.

Diese Ausgestaltung hat den Vorteil, dass wenn ein so ausgestaltetes Halteelement in einen Kanal in einem Knochen mit gleichem Querschnitt eingeführt wird, dies nur in bestimmten Positionen erfolgen kann. Dies bedeutet, dass die Ausnehmung zur Aufnahme des Querbolzens sich in genau definierten Positionen befindet. Dies macht das Einführen des Querbolzens deutlich einfacher und sicherer.

Auch kann durch eine solche Form ein Verdrehen des Implantats in dem Knochenkanal verhindert werden.

In einer weiteren Ausgestaltung verjüngt sich das Halteelement senkrecht zur Schleifenebene in distaler Richtung.

Ein solches Verjüngen verleiht dem Halteelement eine Keilform. Eine solche Keilform vereinfacht das Einsetzen des Halteelements in einen Knochenkanal, da sich das Halteelement durch seine sich verjüngenden Seiten in dem Knochenkanal selbst zentriert.

Es ist sogar möglich, wenn ein keilförmiges Halteelement verwendet wird, auf die Schaffung eines Knochenkanals zu verzichten und das Halteelement direkt in den Knochen einzutreiben, wobei das distale Ende des Keils als Klinge zum Schneiden des Knochenkanals wirkt.

In einer weiteren Ausgestaltung weitet sich die Ausnehmung in Richtung des Querbolzens trichterförmig auf.

Ein solches trichterförmiges Aufweiten der Ausnehmung an ihren Rändern erleichtert das Einführen eines Querbolzens in diese Ausnehmung. Der Querbolzen muss nun nicht mehr vollständig zentriert in die Ausnehmung eingeführt werden, sondern er wird durch die trichterförmigen Ränder beim Auftreffen auf diese Ränder in Richtung des Zentrums der Ausnehmung geführt.

Gemäß der Erfindung besteht das Halteelement aus biodegradierbarem Material.

Implantate aus biodegradierbarem Material werden nach dem Einsetzen in einen Knochen langsam abgebaut und durch nachwachsendes, körpereigenes Gewebe ersetzt. Dies hat den Vorteil, dass nach einer gewissen Zeit das Halteelement vollständig durch körpereigenes Gewebe ersetzt wird, was zu einer besonders sicheren Verbindung zwischen dem Knochen und dem Implantat führt. Degradiert das Halteelement ungleich oder aufgrund anatomischer Besonderheiten des Patienten zu schnell, wird das Implantat dennoch vom Querbolzen vor einem Abziehen gehalten.

Weiterhin sind Halteelemente aus biodegradierbarem Material im Allgemeinen besser verträglich als Halteelemente aus nicht biodegradierbarem Material.

In einer Ausgestaltung dieser Maßnahme sind die Mittel als Rastmechanismus ausgebildet.

Unter einem Rastmechanismus ist hierbei jeder dem Fachmann bekannte Rastmechanismus zu verstehen, wie z.B. eine Kugelraste oder gefederte Rastfinger.

Ein Rastmechanismus hat sich als besonders bevorzugte Ausgestaltung der o.g. Maßnahme gezeigt, da auf der einen Seite das Halteelement fest mit dem Applikator verbunden ist, aber auf der anderen Seite nach dem Sichern des Halteelements mit dem Querbolzen kein zusätzliches Entriegeln mehr notwendig ist, um den Applikator von dem Halteelement zu trennen.

In einer besonderen Ausgestaltung der zuvor genannten Maßnahme besteht der Querbolzen aus biodegradierbarem Material.

Implantate aus biodegradierbarem Material werden nach dem Einsetzen in einen Knochen langsam abgebaut und durch nachwachsendes, körpereigenes Gewebe ersetzt. Dies hat den Vorteil, dass nach einer gewissen Zeit der Querbolzen vollständig durch körpereigenes Gewebe ersetzt wird, was zu einer besonders sicheren Verbindung zwischen dem Knochen und dem Implantat führt.

In einer weiteren Ausgestaltung weist das Instrumentarium ferner einen Applikator zum Einführen des Halteelements in einen Knochen auf.

Das Einführen eines Halteelements zusammen mit einem strangförmigen Implantat in einen Knochen ist ein schwieriger Vorgang, bei dem es zu einem Lösen des Implantats von dem Halteelement kommen kann.

Mittels eines solchen Applikators kann ein fester Zusammenbau aus Halteelement und Applikator geschaffen werden, der dann sicher von einem Operateur manipuliert werden kann. Der Applikator erleichtert somit das Einbringen des Halteelements in den Knochen und ermöglicht eine Korrektur der Position des Halteelements. Dabei kann der Applikator so ausgestaltet sein, dass damit das Halteelement in einen bereits vorgefertigten Knochenkanal eingeführt z.B. eingeschoben wird, oder dass der Applikator bzw. die Kombination aus Applikator und Halteelement den Knochenkanal überhaupt schaffen.

In einer weiteren Ausgestaltung der o.g. Maßnahme weist der Applikator zumindest einen ersten Arm zum Einführen eines Halteelements in einen Knochen und zumindest einen zweiten Arm zum Einführen eines Querbolzens in einen Knochen auf.

Durch diese Maßnahme wird sichergestellt, dass der Querbolzen stets in einer genau definierten Relativposition zu einem an dem Applikator befestigten Halteelement eingeführt wird.

In einer weiteren Ausgestaltung weist der Applikator zwei erste Arme zum Einführen von Halteelementen auf. Dabei sind die beiden ersten Arme so angeordnet, dass Ausnehmungen von an den ersten Armen angebrachten Halteelementen fluchten.

Durch diese Maßnahme können in einem Arbeitsgang zwei Halteelemente in definierter Relativposition beabstandet zueinander in einen Knochen eingebracht werden, die mit einem einzigen Querbolzen gesichert werden.

Das Anbringen von zwei Halteelementen in einer definierten Relativposition hat sich besonders bei der Reparatur von Schäden der Rotatorenmanschette als vorteilhaft erwiesen, da in einem Arbeitsgang zwei Implantate, z.B. in Form von Nähfäden, in den Humeruskopf eingebracht werden können, wodurch großflächig abgelöste Teile einer Rotatorenmanschette wieder sicher mit dem Humeruskopf verbunden werden können.

In einer weiteren Ausgestaltung der o.g. Maßnahme sind die beiden ersten Arme entlang einer Fluchtlinie der Ausnehmungen der an den ersten Armen angebrachten Halteelemente gegeneinander verschiebbar.

Durch die o.g. Maßnahme ist es möglich, den Abstand der ersten Arme und damit die Position der in den Knochen einzubringenden Halteelemente gegeneinander zu verändern und dadurch den Applikator an verschiedene anatomische Gegebenheiten anzupassen.

In einer weiteren Ausgestaltung weist der zumindest eine zweite Arm eine Bohrbuchse auf.

Durch eine solche Bohrbuchse kann vor dem Einbringen des Querbolzens in einer definierten Relativposition zur Ausnehmung des einen an dem ersten Arm befestigten Halteelements eine Bohrung in dem Knochen gesetzt werden, was das Einbringen des Querbolzens deutlich erleichtert.

In einer weiteren Ausgestaltung weist der zumindest eine erste Arm Mittel auf, um diesen lösbar mit einem Halteelement zu verbinden.

Durch diese Maßnahme kann ein Halteelement zunächst fest mit dem Applikator verbunden werden. Dieser Zusammenbau aus Halteelement und Applikator kann dann von einem Operateur manipuliert werden, ohne dass die Gefahr besteht, dass das Halteelement sich vom Applikator löst.

Weiterhin ist es möglich, ein in den Knochen eingebrachtes Halteelement, das noch mit dem Applikator verbunden ist, wieder aus dem Knochen herauszuziehen, wenn der Operateur nicht mit der Lage des Halteelements zufrieden ist.

Ist nun das Halteelement zur Zufriedenheit des Operateurs in dem Knochen eingebracht und der Querbolzen in die Ausnehmung eingebracht, kann der Applikator aus dem Knochenkanal herausgezogen werden, wobei das Halteelement im Knochen verbleibt.

In einer Ausgestaltung dieser Maßnahme sind die Mittel als Rastmechanismus ausgebildet.

Unter einem Rastmechanismus ist hierbei jeder dem Fachmann bekannte Rastmechanismus zu verstehen, wie z.B. eine Kugelraste oder gefederte Rastfinger.

Ein Rastmechanismus hat sich als besonders bevorzugte Ausgestaltung der o.g. Maßnahme gezeigt, da auf der einen Seite das Halteelement fest mit dem Applikator verbunden ist, aber auf der anderen Seite nach dem Sichern des Halteelements mit dem Querbolzen kein zusätzliches Entriegeln mehr notwendig ist, um den Applikator von dem Halteelement zu trennen.

In einer weiteren Ausgestaltung weist das Instrumentarium ferner einen Bohrer zum Bohren eines Kanals für einen Querbolzen auf und insbesondere weist es ferner eine Fräse zum Fräsen eines Kanals für den Querbolzen auf.

Auch wenn es möglich ist, einen Querbolzen allein durch Einschlagen in einen Knochen einzutreiben, ist es nicht immer möglich, den Querbolzen während dieses Einschlagens sauber zu positionieren. Wird nun mittels eines Bohrers oder einer Fräse oder einer Kombination der beiden ein Kanal in dem Knochen geschaffen, wird dadurch ein Weg zum Einführen des Querbolzens geschaffen und ein präzises Einführen des Querbolzens sichergestellt.

In einer weiteren Ausgestaltung der Erfindung ist die Fräse als Hohlfräse ausgebildet.

Die Ausbildung der Fräse als Hohlfräse hat den Vorteil, dass nach dem Fräsen eines Kanals für den Querbolzen dieser durch das Lumen der Hohlfräse hindurchgeführt werden kann, wobei die Hohlfräse als Einführhilfe für den Querbolzen dient.

In einer weiteren Ausgestaltung ist die Fräse mit dem Bohrer koppelbar, wobei der Bohrer in gekoppeltem Zustand vorzugsweise über die Fräse übersteht.

Durch diese Maßnahme kann mit einem einzigen Bohr-/Fräsvorgang ein Kanal geschaffen werden, der zwei verschiedene Durchmesser aufweist. Vorzugsweise weist der erste Kanal einen Durchmesser auf, der größer als der des Querbolzens ist und der zweite Kanal einen Durchmesser, der kleiner ist als der des Querbolzens.

Nach dem Bohr-/Fräsvorgang kann der Bohrer dann aus dem Lumen der Hohlfräse entfernt und der Querbolzen durch das Lumen der Hohlfräse hindurchgeführt werden. Der Querbolzen kann somit durch das Lumen der Hohlfräse als auch durch den Kanal mit dem größeren Durchmesser an den Kanal mit kleinerem Durchmesser herangeführt werden und dann im Presssitz in diesen zweiten Abschnitt des Kanals eingeführt werden.

In einer weiteren Ausgestaltung weist das Instrumentarium ferner einen Einschläger zum Einschlagen eines Kanals für ein Halteelement auf.

Mit einem Einschläger können Kanäle geschaffen werden, die keinen kreisförmigen Querschnitt aufweisen. Ein Kanal, der einen nicht kreisförmigen Querschnitt aufweist, erleichtert das Ausrichten eines Halteelements in diesem Kanal und verhindert ein Verdrehen des Halteelements.

Außerdem weist ein Einschläger keine beweglichen Teile auf, was sowohl seine Herstellung als auch seine Reinigung besonders einfach macht.

In einer weiteren Ausgestaltung weist der Einschläger einen Querschnitt einer Form auf, die einem Querschnitt eines Halteelements entspricht, insbesondere einen Querschnitt, der kleiner ist als der Querschnitt des Halteelements.

Mit einem Einschläger mit einem Querschnitt, der dem des Halteelements entspricht, kann ein Kanal geschaffen werden, der dem des Halteelements entspricht. Somit kann ein Knochenkanal geschaffen werden, in dem das Halteelement in einer definierten Position eingeführt werden kann. Dies erleichtert das Einführen des Querbolzens, da das Halteelement und damit die Ausnehmung des Halteelements sich immer in der gleichen definierten Position befinden.

Ist der Querschnitt des Einschlägers kleiner als der des Halteelements, kann damit ein Kanal in den Knochen eingeschlagen werden, in dem ein Halteelement im Presssitz eingeführt wird. Dadurch wird das Halteelement fest in den Kanal eingebracht. Dieses verhindert Bewegungen des Halteelements, die das Halteelement beschädigen oder den Querbolzen aus seiner Verankerung lösen könnten.

In einer weiteren Ausgestaltung weist der Einschläger einen viereckigen Querschnitt auf.

Mittels eines Einschlägers mit viereckigem Querschnitt kann ein Kanal mit viereckigem Querschnitt eingeschlagen werden. Ein solcher Kanal weist den Vorteil auf, dass wenn ein viereckiges Halteelement eingeführt wird, dieses verdrehsicher in einer definierten Position in den Kanal eingeführt werden kann.

In einer weiteren Ausgestaltung weist der Einschläger an einem distalen Abschnitt zumindest eine Skalierung auf.

Eine solche Skalierung dient dazu, um die Eindringtiefe des Einschlägers und damit die Tiefe des eingeschlagenen Kanals einem Operateur schnell und auf einfache Weise sichtbar zu machen.

In einer weiteren Ausgestaltung ist der Einschläger gabelförmig ausgebildet.

Durch diese Maßnahme können mit einem Einschläger in einem Arbeitsgang zwei oder mehr Kanäle in einen Knochen eingeschlagen werden. Dabei werden die Kanäle in genau definierten Relativpositionen zueinander in den Knochen eingeschlagen.

Diese Maßnahme erweist sich insbesondere in Kombination mit einem Applikator mit zwei oder mehreren Armen zum Einführen eines Halteelements als nützlich, da dadurch zwei oder mehrere Kanäle geschaffen werden können, in die dann in einem Arbeitsgang zwei oder mehrere Halteelemente eingesetzt und diese mit einem einzigen Querbolzen gesichert werden können.

In einer weiteren Ausgestaltung weist das Instrumentarium ferner einen Stößel zum Positionieren eines Querbolzens in einem Knochen auf.

Ein solcher Stößel weist einen Schaft auf, dessen Querschnitt dem Querschnitt eines Querbolzens entspricht und wird dazu verwendet, einen Querbolzen präzise in einem Knochen zu positionieren. Insbesondere wenn der Querbolzen in einen Kanal mit geringerem Durchmesser eingeführt wird, kann der Stößel dazu verwendet werden, den Querbolzen in diesen Kanal einzupressen oder einzuschlagen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Figur 1: eine perspektivische Ansicht eines Halteelements;
- Figur 2: eine Draufsicht auf das proximale Ende des Halteelements von Figur 1;
- Figur 3: eine Seitenansicht des Halteelements von Figur 2;
- Figur 4: einen Schnitt durch das Halteelement von Figur 3 entlang der Linie IV-IV;
- Figur 5: eine Explosionsdarstellung eines Instrumentariums zum Fixieren eines strangförmigen Implantats in einem Knochen;
- Figur 6: den Einschläger des Instrumentariums von Figur 5;
- Figur 6a: einen schnitt durch den Einschläger von Figur 6 entlang der Linie VIa-VIa;
- Figur 7: den Applikator des Instrumentariums von Figur 5;
- Figur 8: eine vergrößerte Darstellung eines Abschnitts des Applikators von Figur 7 zusammen mit dem Halteelement von Figur 1;
- Figur 9: den Abschnitt von Figur 8, wobei das Halteelement an dem Abschnitt angebracht ist;
- Figur 10: die Hohlfräse des Instrumentariums von Figur 5;
- Figur 11: den Bohrer des Instrumentariums von Figur 5;
- Figur 12: eine schematische Darstellung eines Schnitts durch einen Humeruskopf, wobei ein Halteelement eingesetzt ist; und
- Figur 13: eine schematische Darstellung eines durch ein Halteelement gesicherten Abschnitts einer Rotatormanschette.

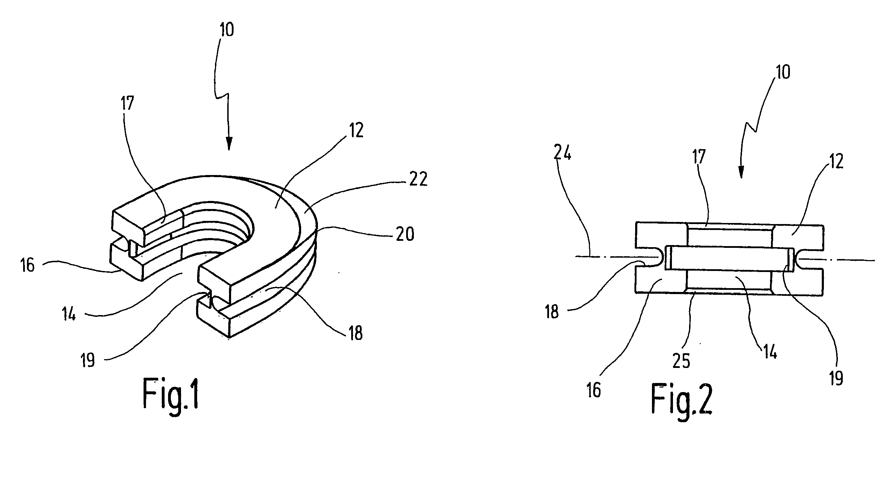

In Figur 1 ist ein Halteelement in seiner Gesamtheit mit der Bezugsziffer 10 bezeichnet.

Das Halteelement 10 weist einen U-förmigen Körper 12 auf, in dessen Mitte eine Ausnehmung 14 angeordnet ist.

Die Ausnehmung 14 öffnet sich in Richtung eines proximalen Endes 16 des Körpers 12. Eine Kante 17 der Ausnehmung 14 ist abgeschrägt, wodurch der Rand der Ausnehmung 14 ein sich trichterförmig aufweitendes Profil aufweist.

In die äußere Konturfläche des U-förmigen Körpers 12 ist umfänglich eine Nut 18 eingebracht. Die Nut 18 weist ein ebenfalls U-förmiges Profil auf. Diese Nut 18 bildet eine Führung für ein strangförmiges Implantat.

In die Innenkontur des Körpers 12 ist eine Nut 19 eingebracht. Diese Nut 19 weist ein viereckiges Profil auf. Diese Nut 19 dient zur Verbindung des Halteelements 10 mit einem Applikator.

An einem distalen Ende 20 des Körpers 12 befindet sich ein sichelförmiger Abschnitt 22. Dieser sichelförmige Abschnitt 22 ist abgeschrägt, wodurch die Dicke des Körpers 12 im Bereich des sichelförmigen Abschnitts 22 in distaler Richtung abnimmt.

In Figur 2 ist eine Draufsicht auf das proximale Ende 16 des Körpers 12 des Halteelements 10 dargestellt.

Die strichpunktierte Linie zeigt dabei eine Schleifenebene 24 an, die durch die Nut 18 definiert wird.

In dieser Darstellung wird sichtbar, dass die Ausnehmung 14 auf der der Kante 17 gegenüberliegenden Seite eine weitere abgeschrägte Kante 25 aufweist. Somit weist die Ausnehmung 14 auf beiden Seiten ein trichterförmiges Profil auf.

In dieser Darstellung ist noch einmal das U-förmige Profil der äußeren Nut 18 und das viereckige Profil der inneren Nut 19 sichtbar.

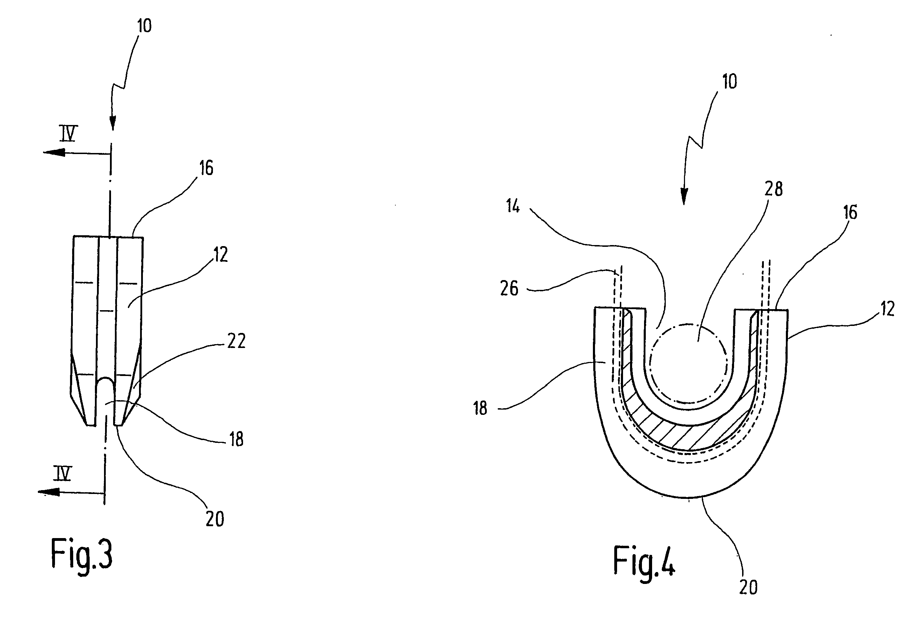

In Figur 3 ist eine Seitenansicht auf das Halteelement 10 dargestellt. Hier wird sichtbar, dass sich der Körper 12 des Halteelements 10 in Richtung des distalen Endes 20 in dem sichelförmigen Bereich 22 verjüngt, wodurch das distale Ende 20 ein keilförmiges Profil aufweist.

In dieser Figur ist ebenfalls sichtbar, dass die Nut 18 im Bereich des distalen Endes 20 des Körpers 12 besonders tief ist, wodurch eine besonders sichere Führung eines Implantats gewährleistet wird.

In Figur 4 ist ein Schnitt durch ein Halteelement 10 entlang der Linie IV-IV von Figur 3 dargestellt. Weiterhin ist durch die gestrichelte Linie ein strangförmiges Implantat 26 und durch die strichpunktierte Linie ein Querbolzen 28 dargestellt.

Das Implantat 26 wird dabei durch die Nut 18 distalseitig um den Querbolzen 28 herumgeführt. Hier wird ersichtlich, dass die Nut 18 im Bereich des distalen Endes 20 des Halteelements deutlich tiefer ist als im Bereich des proximalen Endes 16.

Der Querbolzen 28 ist in der Ausnehmung 14 des Halteelements 10 aufgenommen.

Würde es nun unter einer Zugbelastung des Implantats 26 in Richtung des distalen Endes 16 des Halteelements 10 zu einem Versagen des Körpers 12 des Halteelements 10 kommen, ist ersichtlich, dass das Implantat 26 noch immer durch den Querbolzen 28 gesichert ist.

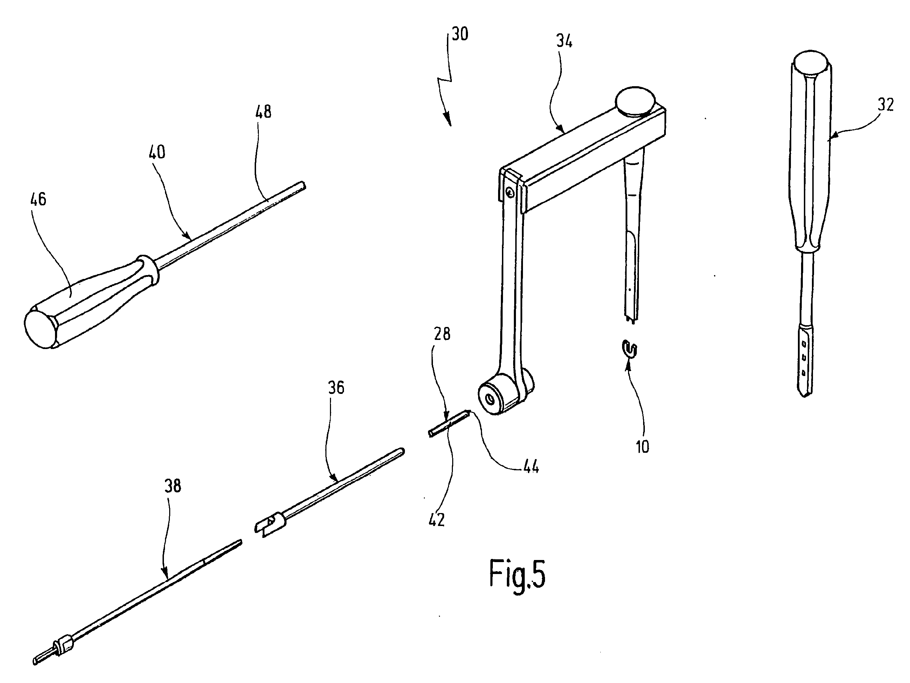

In Figur 5 ist ein Instrumentarium zum Fixieren eines strangförmigen Implantats in einem Knochen in seiner Gesamtheit mit der Bezugsziffer 30 bezeichnet.

Das Instrumentarium 30 weist ein Halteelement 10 auf, wie dieses bereits in Figuren 1 bis 4 dargestellt wurde.

Das Instrumentarium 30 weist weiterhin einen Querbolzen 28 auf, wie dieser bereits in Figur 4 dargestellt wurde.

Ferner weist das Instrumentarium 30 einen Einschläger 32, einen Applikator 34, eine Hohlfräse 36, einen Bohrer 38 und einen Stößel 40 auf.

Der Querbolzen 28 weist einen zylindrischen Körper 42 auf, der sich in distaler Richtung zur Spitze 44 verjüngt.

Der Stößel 40 weist einen Griff 46 und einen Schaft 48 auf. Der Schaft 48 weist dabei den gleichen Querschnitt auf wie der Körper 42 des Querbolzens 28. Der Griff 46 des Stößels 40 ist dabei so ausgelegt, dass der Stößel 40 dazu verwendet werden kann, einen Querbolzen 28 mittels eines Hammers in einem Knochen zu positionieren.

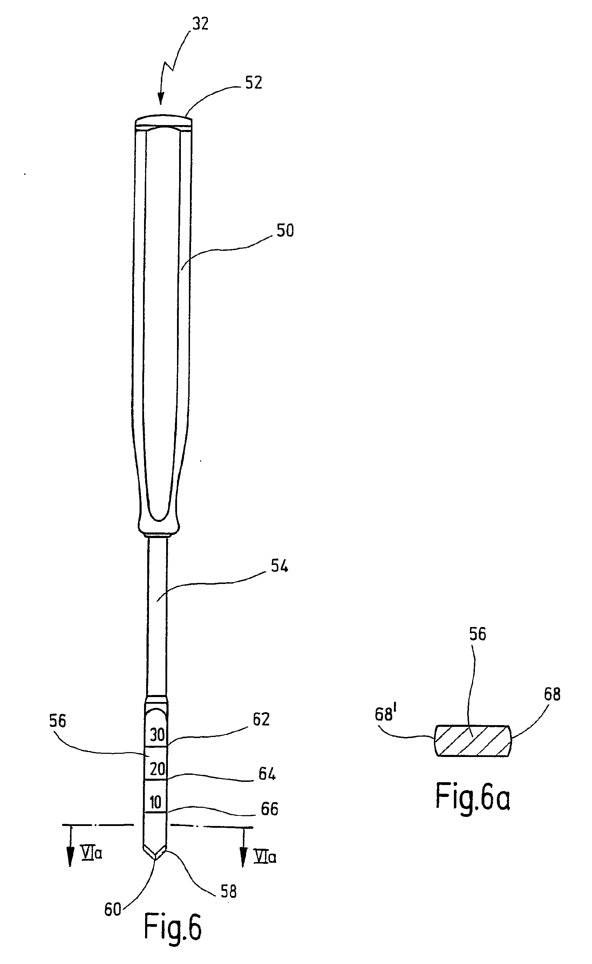

In Figur 6 ist der Einschläger 32 des Instrumentariums 30 von Figur 5 in größtem Detail dargestellt.

Der Einschläger 32 weist einen Griff 50 auf. Der Griff 50 weist ein proximales Ende 52 auf, das so ausgestaltet ist, dass der Einschläger 32 mittels eines Hammers in einen Knochen eingetrieben werden kann.

Distal des Griffs 50 schließt sich ein Schaft 54 an, der distal in einen Meißelabschnitt 56 übergeht. An seinem distalen Ende weist der Meißelabschnitt 56 Klingen 58 auf, die zu einer Spitze 60 zusammenlaufen. Die Klingen 58 in Kombination mit der Spitze 60 schneiden während des Einschlagens des Einschlägers 32 einen Knochenkanal in einen Knochen.

Der Meißelabschnitt 56 weist weiterhin Skalierungen 62, 64 und 66 auf. Diese Skalierungen 62, 64 und 66 erlauben eine einfache Kontrolle der Eindringtiefe des Einschlägers 32 in einen Knochen.

In Figur 6a ist ein Schnitt durch den Einschläger 32 entlang der Linie VIa-VIa von Figur 6 dargestellt.

Aus dieser Darstellung wird deutlich, dass der Meißelabschnitt 56 des Einschlägers 32 einen im Wesentlichen rechteckigen Querschnitt aufweist. Somit kann mit dem Einschläger 32 ein Kanal in einen Knochen eingeschlagen werden, der einen im Wesentlichen rechteckigen Querschnitt aufweist. Dieser rechteckige Querschnitt des Knochenkanals in Kombination mit dem rechteckigen Abschnitt des proximalen Endes 16 des Halteelements 10 ermöglicht es, das Halteelement in einer genau definierten Position in den Knochenkanal einzuführen.

Der Querschnitt des Meißelabschnitts 56 ist dabei etwas kleiner als der des proximalen Endes 16 des Halteelements 10, so dass mit dem Einschläger 32 ein Kanal geschaffen werden kann, in dem das Halteelement 10 im Presssitz eingebracht wird.

Die schmalen Seiten 68, 68' des Meißelabschnitts 56 sind konvex ausgebildet. Dies ermöglicht es, ein Halteelement 10 mit einem Implantat 26 einzubringen, das in gewissem Maße über die Nut 18 des Halteelements 10 übersteht.

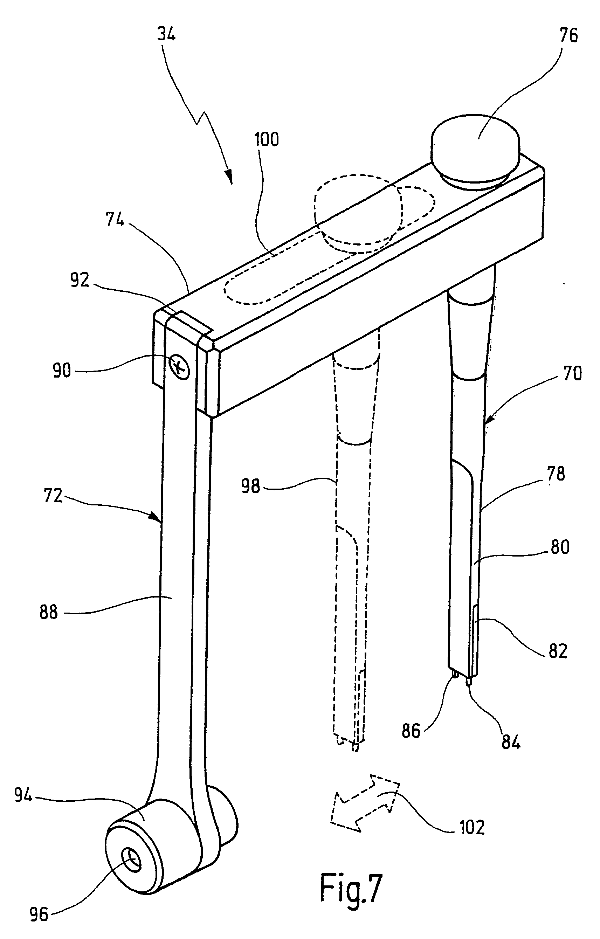

In Figur 7 ist der Applikator 34 des Instrumentariums 30 von Figur 5 dargestellt.

Der Applikator 34 weist einen ersten Arm 70 zum Einbringen eines Halteelements 10 in einen Knochen und einen zweiten Arm 72 zum Einbringen eines Querbolzens 28 in einen Knochen auf. Der erste Arm 70 und der zweite Arm 72 sind dabei durch das Verbindungselement 74 miteinander verbunden.

An seinem proximalen Ende weist der Arm 70 einen Knauf 76 auf, der so ausgebildet ist, dass der Arm 70 mittels eines Hammers in einen Knochenkanal eingetrieben werden kann.

Der distale Abschnitt 78 des Arms 70 weist einen in etwa rechteckigen Querschnitt auf. Der Querschnitt des distalen Abschnitts 78 entspricht dabei dem Querschnitt des Meißelabschnitts 56 des Einschlägers 32.

In einer schmalen Seite 80 des distalen Abschnitts 78 des Arms 70 ist eine Ausnehmung 82 eingebracht. Diese Ausnehmung 82 dient dazu, ein Implantat 26 entlang des distalen Abschnitts 78 zu führen, ohne dass dieses dabei über die Seite 80 hinaussteht.

Am Ende des distalen Abschnitts 78 des Arms 70 sind zwei gefederte Rastfinger 84 und 86 angebracht, die dazu vorgesehen sind, ein Halteelement 10 mit dem Arm 70 zu verbinden.

Der zweite Arm 72 weist einen im Wesentlichen rechteckigen Körper 88 auf, der an seinem proximalen Ende mittels der Schraube 90 mit dem Verbindungselement 74 verbunden ist. Hierbei kommt der Körper 88 des Arms 72 in einer Ausnehmung 92 des Verbindungselements 74 zu liegen, wodurch der Arm 72 verdrehsicher mit dem Verbindungselement 74 verbunden ist.

An seinem distalen Ende weist der Arm 72 eine Bohrbuchse 94 auf. Diese Bohrbuchse weist ein Lumen 96 auf, das senkrecht zum Körper 88 des Arms 72 steht. Das Lumen 96 der Bohrbuchse 94 ist dabei so angeordnet, dass es mit einer Ausnehmung 14 eines an dem Arm 70 angebrachten Halteelements 10 fluchtet.

Durch die gestrichelten Linien ist eine zweite Ausführungsform des Applikators 34 dargestellt. Diese zweite Ausführungsform weist einen weiteren Arm 98 auf, der dem ersten Arm 70 entspricht.

Dieser Arm 98 ist entlang eines Langlochs 100 im Verbindungselement 74 in Richtung des Doppelpfeils 102 verschiebbar. Der Arm 98 ist hierbei so angeordnet, dass eine Ausnehmung 14 eines an dem Arm 98 angebrachten Halteelements 10 mit einer Ausnehmung 14 eines an dem Arm 70 angebrachten Halteelements 10 fluchtet. Die Ausnehmungen der beiden Halteelemente fluchten wiederum mit dem Lumen 96 der Bohrbuchse 94.

Durch einen so ausgestalteten Applikator können zwei Halteelemente 10 in definierter Relativposition zueinander in einen Knochen eingebracht und dort mit einem einzigen Querbolzen 28 gesichert werden.

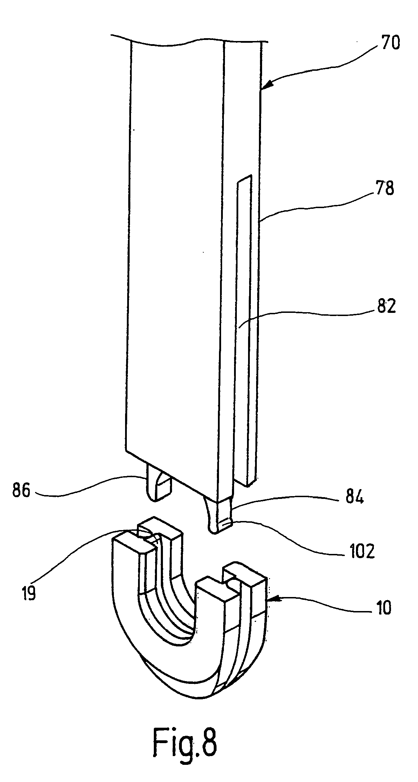

Figur 8 zeigt eine vergrößerte Darstellung des distalen Abschnitts 78 des ersten Arms 70 des Applikators 34. Weiterhin ist das Halteelement 10 dargestellt.

In dieser Darstellung wird deutlich, dass die Rastfinger 84 und 86 so angeordnet sind, dass sie in die Nut 19 in der Innenkontur des Halteelements 10 eingreifen können.

Der Haltefinger 84 weist dabei eine Wulst 102 auf, die die sichere Verbindung zwischen dem Rastfinger 84 und der Nut 19 sicherstellt. Der Haltefinger 86 weist eine ebensolche Wust auf, die in dieser Darstellung allerdings nicht sichtbar ist.

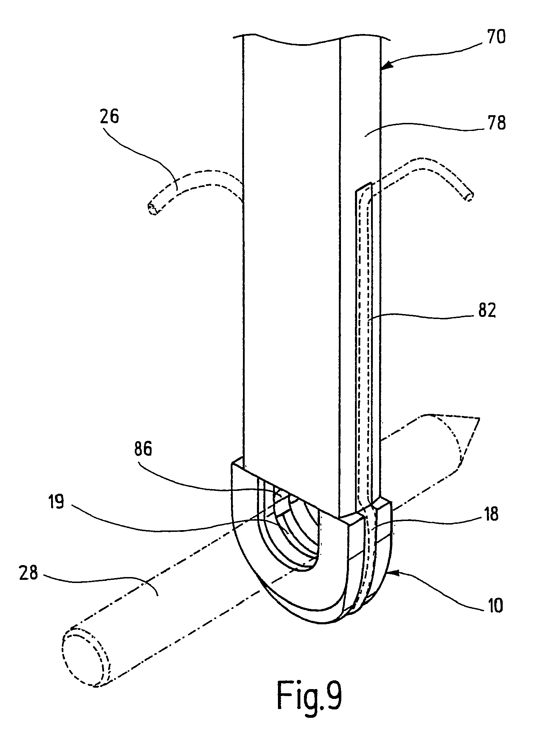

In Figur 9 ist ein proximaler Abschnitt 78 des Arms 70 des Applikators 34 dargestellt, wobei ein Halteelement 10 am distalen Ende des Arms 70 angebracht ist. Der Rastfinger 86 greift hier in die Nut 19 des Halteelements 10 ein, wodurch eine sichere Verbindung zwischen dem Halteelement 10 und dem Arm 70 gewährleistet wird.

Durch die gestrichelten Linien ist ein strangförmiges Implantat 26 dargestellt, das den distalen Abschnitt 78 des Arms 70 und das Halteelement 10 umschlingt, wobei es in der Nut 18 bzw. in der Ausnehmung 82 zu liegen kommt.

Durch die strichpunktierten Linien ist ein Querbolzen 28 dargestellt, der durch die Ausnehmung 14 des Halteelements 10 hindurchgeführt ist, wobei das proximale Ende der Ausnehmung 14 durch das distale Ende des Arms 70 begrenzt wird.

In dem durch die gestrichelten/strichpunktierten Linien dargestellten Zustand ist das Halteelement 10 und das Implantat 26 durch den Querbolzen 28 gesichert und der Arm 70 kann von dem Halteelement 10 abgezogen werden.

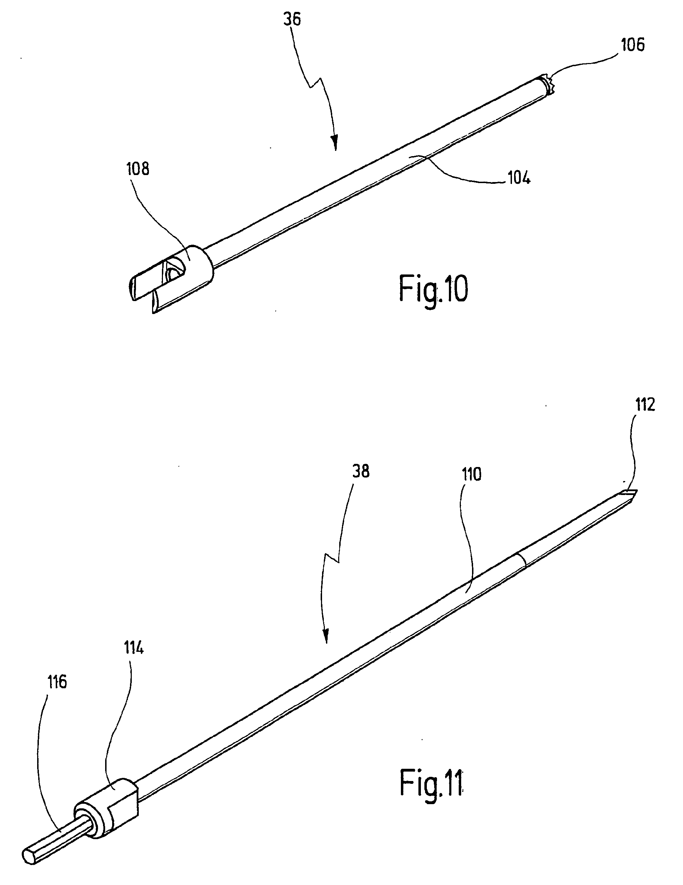

In Figur 10 ist die Hohlfräse 36 des Instrumentariums 30 von Figur 5 dargestellt.

Die Hohlfräse 36 weist einen Hohlschaft 104 auf, wobei der lichte Innendurchmesser des Hohlschafts 104 in etwa dem Durchmesser des Körpers 12 des Querbolzens 28 entspricht. An einem distalen Ende weist der Hohlschaft 104 einen ringförmigen Fräskopf 106 auf. An einem proximalen Ende weist der Hohlschaft 104 ein Kopplungselement 108 auf, das dazu vorgesehen ist, die Fräse 36 mit dem Bohrer 38 zu koppeln.

In Figur 11 ist der Bohrer 38 des Instrumentariums 30 von Figur 5 dargestellt.

Der Bohrer 38 weist dabei einen Schaft 110 auf, dessen Durchmesser in etwa dem lichten Innendurchmesser des Hohlschafts 104 der Fräse 36 entspricht. An einem distalen Ende des Schafts 110 ist eine Klinge 112 zum Bohren eines Knochenkanals angebracht. An einem proximalen Ende des Schafts schließt sich ein Kopplungselement 114 an, das dazu vorgesehen ist, den Bohrer 38 mit der Fräse 36 in Wirkverbindung zu bringen.

Proximal an das Kopplungselement 114 schließt sich ein Abschnitt 116 an, der einen sechseckigen Querschnitt aufweist und der dazu dient, den Bohrer 38 mit einer Bohrmaschine zu verbinden.

Der Bohrer 38 kann mit der Fräse 36 gekoppelt werden, indem der Schaft 110 des Bohrers 38 in den Hohlschaft 104 der Fräse 36 eingeführt wird. Das Kopplungselement 114 des Bohrers 38 kommt dabei in dem Kopplungselement 108 der Fräse 36 zu liegen, wodurch eine Wirkverbindung zwischen dem Bohrer 38 und der Fräse 36 geschaffen wird.

In dem zusammengebauten Zustand steht ein Teil des Schafts 110 des Bohrers 38 über das distale Ende des Schafts 104 der Fräse 36 über. Durch diesen Zusammenbau kann ein Kanal gebohrt werden, der zwei Abschnitte mit unterschiedlichem Durchmesser aufweist.

Nach Beendigung des Bohr-/Fräsvorgangs kann der Bohrer 38 aus dem rohrförmigen Schaft 104 der Fräse 36 entnommen werden, wobei die Fräse 36 noch im Knochen verbleibt. In einem zweiten Arbeitsschritt kann dann ein Querbolzen 28 durch den Hohlschaft 104 der Fräse 36 in den Knochen eingeführt werden.

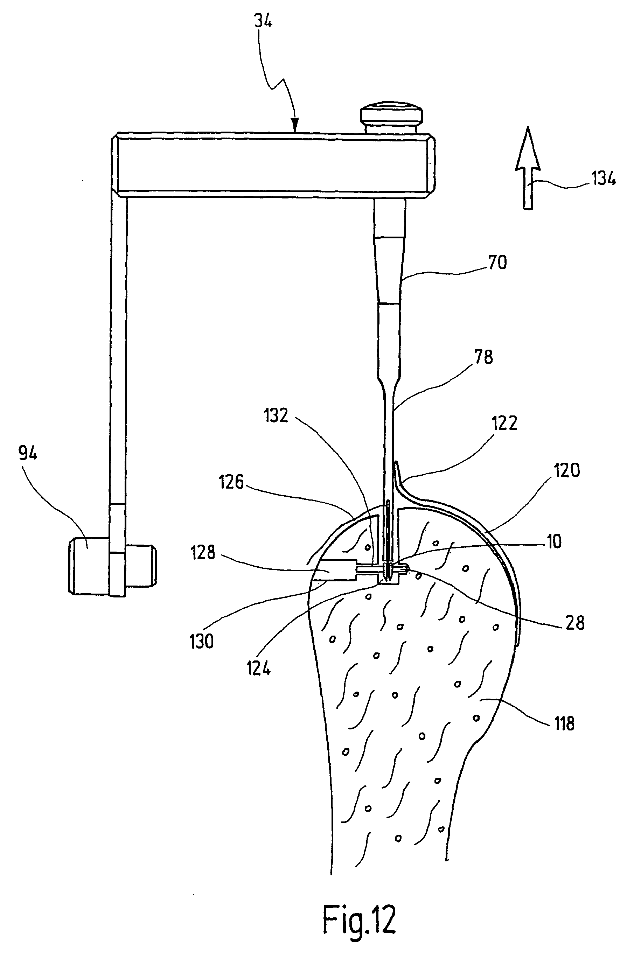

In Figur 12 ist schematisch ein Schnitt durch einen Humeruskopf 118 dargestellt.

An der Außenseite des Humeruskopfes 118 befindet sich eine Rotatorenmanschette 120, wobei diese einen Abschnitt 122 aufweist, der sich von dem Humeruskopf 118 abgelöst hat und wieder mit diesem verbunden werden soll. Hierzu wurde mittels des Einschlägers 32 und eines Hammers ein erster Kanal 124 in den Humeruskopf 118 eingeschlagen. In diesen Kanal 124 wurde der Arm 70 des Applikators 34, an dessen distalen Ende das Halteelement 10 angebracht ist, eingeführt. Ein Implantat in Form eines Fadens 126 umschlingt dabei das Halteelement 10 und einen Teil des distalen Abschnitts 78 des Arms 70.

Mittels des Bohrers 38 und der Fräse 36 wurde dann ein zweiter Kanal 128 in den Humeruskopf 118 gebohrt/gefräst. Die Bohrbuchse 94 des Applikators 34 stellte dabei sicher, dass der Kanal 128 mit der Ausnehmung 14 des Implantats 10 fluchtet. Der zweite Kanal 128 weist dabei einen ersten Abschnitt 130 mit einem größeren Durchmesser und einen zweiten Abschnitt 132 mit einem kleineren Durchmesser auf. Nach dem Bohr-/Fräsvorgang wurde der Bohrer 38 aus dem Schaft der Fräse 36 entnommen.

Der Querbolzen 28 wurde durch den Schaft 104 der Fräse 36 in den Kanal 128 eingeführt und mittels des Stößels 40 in dem zweiten Abschnitt 132 des zweiten Kanals 128 positioniert. Der Querbolzen sichert somit das Implantat 10 und damit den Faden 126 im Humeruskopf 118.

Der Applikator 34 kann jetzt in Richtung des Pfeils 134 aus dem Humeruskopf 118 abgezogen werden und der Abschnitt 122 der Rotatormanschette 120 kann mittels des Fadens 126 wieder mit dem Humeruskopf 118 verbunden werden.

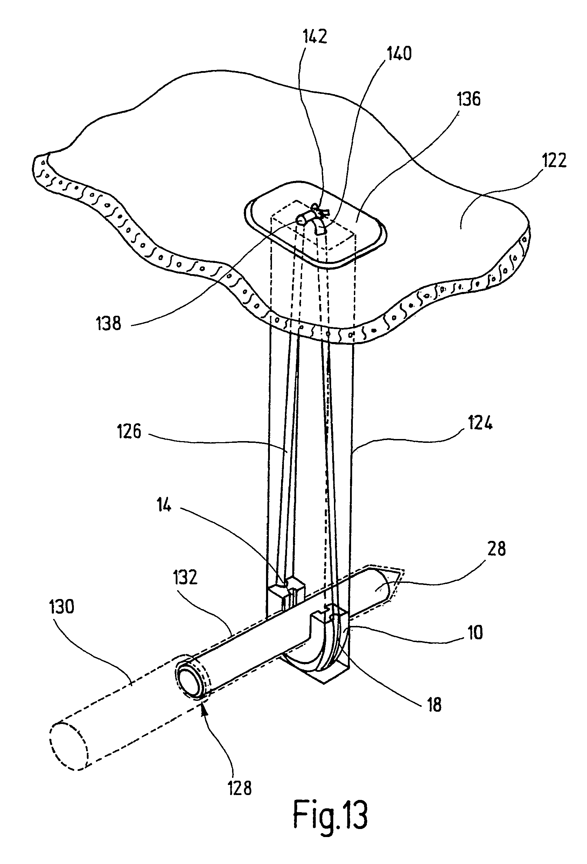

In Figur 13 ist schematisch der Abschnitt 122 der Rotatormanschette dargestellt, der mittels des Halteelements 10, des Querbolzens 28 und des Fadens 126 wieder mit dem Humeruskopf 118 verbunden wurde.

Das Halteelement 10 sitzt im Presssitz in dem Kanal 124, wobei der Kanal 124 im Wesentlichen den gleichen Querschnitt aufweist wie das Halteelement 110, dieser allerdings geringfügig kleiner ist.

Das Halteelement 10 wird durch den Querbolzen 28 gesichert, der in den durch die gestrichelten Linien dargestellten zweiten Kanal 128 eingeführt ist, wobei der Querbolzen 28 vollständig von dem Abschnitt 130 mit großem Durchmesser des zweiten Kanals 128 in den Kanal 132 mit kleinem Durchmesser des zweiten Kanals 128 überführt wurde und in diesen Abschnitt 132 im Presssitz eingebracht ist. Der Querbolzen 28 kommt dabei in der Ausnehmung 14 des Halteelements 10 zu liegen.

Der Faden 126 umschlingt dabei den Querbolzen 28 und das Halteelement 10, wobei der Faden 126 in der Nut 18 des Halteelements 10 zu liegen kommt. Der Faden 126 ist somit gegenüber einem Verschieben entlang der Mittelachse des Bolzens 28 gesichert in dem Kanal 124 angeordnet.

Würde es nun zu einem Versagen des Halteelements 10 kommen, wäre der Faden 126 noch immer durch den Querbolzen 28 gesichert.

Die beiden Enden des Fadens 126 wurden durch den zu befestigenden Abschnitt 122 der Rotatormanschette hindurchgeführt und, mittels der Platte 136 gegen Ausreißen gesichert, angebracht. Die beiden Enden des Fadens 126 wurden dazu durch die Löcher 138 und 140 der Platte 136 durchgeführt und mittels des Knotens 142 miteinander verbunden.

Die Platte 136 dient dazu, ein Zerschneiden der Rotatormanschette 122 durch den Faden 126 zu verhindern. Somit ist der Abschnitt 122 der Rotatormanschette wieder sicher mit dem Humeruskopf 118 verbunden.

Es ist auch möglich, den Faden bereits vorgefertigt als geschlossenen Ringfaden einzusetzen. Der ringförmige Faden wird als Doppelstrang um den abgelösten Abschnitt der Rotatorenmanschette gelegt und einmal durch sich selbst durchgezogen. Dadurch ist der Faden mit dem zu fixierenden Abschnitt bereits verbunden.

In die verbleibende abstehende Schleife wird das Halteelement eingefädelt und in den Knochen eingetrieben, wobei die Größe dieser Schleife die Eintreibtiefe bestimmt.

## Patentansprüche

1. Instrumentarium zum Fixieren eines strangförmigen Implantats (26) in einem Knochen, mit einem im Knochen einsetzbaren Halteelement (10) sowie einem Querbalken (28), wobei das Halteelement (10) eine Ausnehmung (14) zur Aufnahme des Querbolzens (28) sowie Mittel, um es lösbar mit einem Applikator (34) zu verbinden, aufweist und ferner zur Fixierung des Implantats (26) dient, wobei der Querbolzen (28) das Halteelement (10) in dem Knochen verankert und wobei das Halteelement (10) aus einem biodegradierbarem Material besteht, **dadurch gekennzeichnet, dass** das Halteelement (10) eine Führung zur Aufnahme des Implantats (26) aufweist, die so ausgebildet ist, dass das Implantat (26) von dem Halteelement (10) distalseitig schleifenartig um den Querbolzen (28) herumführbar ist.

2. Instrumentarium nach Anspruch 1, **dadurch gekennzeichnet, dass** die Führung als offene Führung ausgebildet ist.

3. Instrumentarium nach Anspruch 1, **dadurch gekennzeichnet, dass** die Führung als geschlossene Führung ausgebildet ist.

4. Instrumentarium nach einem der Ansprüche 1 bis 3 **dadurch gekennzeichnet, dass** eine Mittelachse eines in die Ausnehmung (14) eingeführten Querbolzens (28) senkrecht auf einer durch die Führung definierten Schleifenebene (24) steht.

5. Instrumentarium nach einem der Ansprüche 1 bis 4, **dadurch gekennzeichnet, dass** das Halteelement (10) U-förmig ausgebildet ist.

6. Instrumentarium nach einem der Ansprüche 1 bis 5, **dadurch gekennzeichnet, dass** die Führung die Ausnehmung (14) um zumindest 180° umschlingt.

7. Instrumentarium nach einem der Ansprüche 1 bis 6, **dadurch gekennzeichnet, dass** ein proximales Ende (16) des Halteelements (10) einen viereckigen Querschnitt aufweist.

8. Instrumentarium nach einem der Ansprüche 1 bis 7, **dadurch gekennzeichnet, dass** sich das Halteelement (10) senkrecht zur Schleifenebene (24) in distaler Richtung verjüngt.

9. Instrumentarium nach einem der Ansprüche 1 bis 8, **dadurch gekennzeichnet, dass** sich die Ausnehmung (14) in Richtung des Querbolzens (28) trichterförmig aufweitet.

10. Instrumentarium nach einem der Ansprüche 1 bis 9, **dadurch gekennzeichnet, dass** die Mittel als Rastmechanismus ausgebildet sind.

11. Instrumentarium nach einem der Ansprüche 1 bis 10, **dadurch gekennzeichnet, dass** es ferner einen Applikator (34) zum Einführen des Halteelements (10) in einen Knochen aufweist.

12. Instrumentarium nach Anspruch 11, **dadurch gekennzeichnet, dass** der Applikator (34) zumindest einen ersten Arm (70, 98) zum Einführen eines Halteelements (10) in einen Knochen und zumindest einen zweiten Arm (72) zum Einführen eines Querbolzens (28) in einen Knochen aufweist.

13. Instrumentarium nach Anspruch 12, **dadurch gekennzeichnet, dass** der Applikator (34) zwei erste Arme (70, 98) zum Einführen von Halteelementen (10) aufweist.

14. Instrumentarium nach Anspruch 13, **dadurch gekennzeichnet, dass** die beiden ersten Arme (70, 98) so angeordnet sind, dass Ausnehmungen (14) von an den ersten Armen (70, 98) angebrachten Halteelementen (10) fluchten.

15. Instrumentarium nach Anspruch 14, **dadurch gekennzeichnet, dass** die beiden ersten Arme (70, 98) entlang einer Fluchtlinie der Ausnehmungen (14) der an den ersten Armen (70, 98) angebrachten Halteelementen (10) gegeneinander verschiebbar sind.

16. Instrumentarium nach einem der Ansprüche 12 bis 15, **dadurch gekennzeichnet, dass** der zumindest eine zweite Arm (72) eine Bohrbuchse (94) aufweist.

17. Instrumentarium nach einem der Ansprüche 12 bis 16, **dadurch gekennzeichnet, dass** der zumindest eine erste Arm Mittel (70, 98) aufweist, um diesen lösbar mit einem Halteelement (10) zu verbinden.

18. Instrumentarium nach Anspruch 17, **dadurch gekennzeichnet, dass** die Mittel als Rastmechanismus ausgebildet sind.

19. Instrumentarium nach einem der Ansprüche 1 bis 18, **dadurch gekennzeichnet, dass** es ferner einen Bohrer (38) zum Bohren eines Kanals für einen Querbolzen (28) aufweist.

20. Instrumentarium nach Anspruch 19, **dadurch gekennzeichnet, dass** es ferner eine Fräse (36) zum Fräsen eines Kanals für einen Querbolzen (28) aufweist.

21. Instrumentarium nach Anspruch 20, **dadurch gekennzeichnet, dass** die Fräse als Hohlfräse (36) ausgebildet ist.

22. Instrumentarium nach Anspruch 21, **dadurch gekennzeichnet, dass** die Fräse (36) mit dem Bohrer (38) koppelbar ist.

23. Instrumentarium nach Anspruch 22, **dadurch gekennzeichnet, dass** der Bohrer (38) im gekoppelten Zustand über die Fräse (36) übersteht.

24. Instrumentarium nach einem der Ansprüche 1 bis 23, **dadurch gekennzeichnet, dass** es ferner einen Einschläger (32) zum Einschlagen eines Kanals für ein Halteelement (10) aufweist.

25. Instrumentarium nach Anspruch 24, **dadurch gekennzeichnet, dass** der Einschläger (32) einen Querschnitt einer Form aufweist, die einem Querschnitt eines Halteelements (10) entspricht.

26. Instrumentarium nach Anspruch 25, **dadurch gekennzeichnet, dass** der Querschnitt des Einschlägers (32) kleiner ist als der Querschnitt des Halteelements (10).

27. Instrumentarium nach einem der Ansprüche 24 bis 26, **dadurch gekennzeichnet, dass** der Einschläger (32) einen viereckigen Querschnitt aufweist.

28. Instrumentarium nach einem der Ansprüche 24 bis 27, **dadurch gekennzeichnet, dass** der Einschläger (32) an einem distalen Abschnitt zumindest eine Skalierung (62, 64, 66) aufweist.

29. Instrumentarium nach einem der Ansprüche 24 bis 28, **dadurch gekennzeichnet, dass** der Einschläger (32) gabelförmig ausgebildet ist.

30. Instrumentarium nach einem der Ansprüche 1 bis 29, **dadurch gekennzeichnet, dass** es ferner einen Stößel (40) zum Positionieren eines Querbolzens (28) in einem Knochen aufweist.

## Claims

1. An instrument set for fixing a cord-like implant (26) in a bone, with a retaining element (10) that can be inserted into the bone as well as a transverse pin (28), which retaining element (10) comprises a recess (14) for receiving the transverse pin (28) as well as means for connecting it releasably to an applicator (34) and is also used to fix the implant (26), whereby the transverse pin (28) anchors the retaining element (10) in the bone and whereby the retaining element (10) is made of biodegradable material, **characterized in that** the retaining element (10) comprises a guide for receiving the implant (26), the guide being configured in such a way that the implant (26) can be guided in a loop formation around the transverse pin (28) distally from the retaining element (10).

2. The instrument set according to claim 1, **characterized in that** the guide is configured as an open guide.

3. The instrument set according to claim 1, **characterized in that** the guide is configured as a closed guide.

4. The instrument set according to any one of claims 1 to 3, **characterized in that** a central axis of a transverse pin (28) inserted into the recess (14) is perpendicular to a loop plane (24) defined by the guide.

5. The instrument set according to any one of claims 1 to 4, **characterized in that** the retaining element (10) has a U-shaped configuration.

6. The instrument set according to any one of claims 1 to 5, **characterized in that** the guide extends round the recess (14) by at least 180°.

7. The instrument set according to any one of claims 1 to 6, **characterized in that** a proximal end (16) of the retaining element (10) has a quadrangular cross section.

8. The instrument set according to any one of claims 1 to 7, **characterized in that** the retaining element (10) narrows in the distal direction perpendicular to the loop plane (24).

9. The instrument set according to any one of claims 1 to 8, **characterized in that** the recess (14) widens in a funnel shape in the direction of the transverse pin (28).

10. The instrument set according to any one of claims 1 to 9, **characterized in that** the means are configured as a locking mechanism.

11. The instrument set according to any one of claims 1 to 10, **characterized in that** it additionally comprises an applicator (34) for inserting the retaining element (10) into a bone.

12. The instrument set according to claim 11, **characterized in that** the applicator (34) comprises at least one first arm (70, 98) for inserting a retaining element (10) into a bone, and at least one second arm (72) for inserting a transverse pin (28) into a bone.

13. The instrument set according to claim 12, **characterized in that** the applicator (34) comprises two first arms (70, 98) for inserting retaining elements (10).

14. The instrument set according to claim 13, **characterized in that** the two first arms (70, 98) are arranged such that recesses (14) of retaining elements (10) arranged on the first arms (70, 98) are in alignment.

15. The instrument set according to claim 14, **characterized in that** the two first arms (70, 98) are displaceable relative to one another along an alignment line of the recesses (14) of the retaining elements (10) arranged on the first arms (70, 98).

16. The instrument set according to any one of claims 12 to 15, **characterized in that** the at least one second arm (72) comprises a drill bushing (94).

17. The instrument set according to any one of claims 12 to 16, **characterized in that** the at least one first arm comprises means (70, 98) for connecting it releasably to a retaining element (10).

18. The instrument set according to claim 17, **characterized in that** the means are configured as a locking mechanism.

19. The instrument set according to any one of claims 1 to 18, **characterized in that** it also comprises a drill (38) for drilling a channel for a transverse pin (28).

20. The instrument set according to claim 19, **characterized in that** it also comprises a milling cutter (36) for cutting a channel for a transverse pin (28).

21. The instrument set according to claim 20, **characterized in that** the milling cutter is configured as a hollow milling cutter (36).

22. The instrument set according to claim 21, **characterized in that** the milling cutter (36) can be coupled to the drill (38).

23. The instrument set according to claim 22, **characterized in that** the drill (38) protrudes beyond the milling cutter (36) in the coupled state.

24. The instrument set according to any one of claims 1 to 23, **characterized in that** it also comprises an impactor (32) for creating a channel for a retaining element (10).

25. The instrument set according to claim 24, **characterized in that** the impactor (32) has a cross section with a shape corresponding to a cross section of a retaining element (10).

26. The instrument set according to claim 25, **characterized in that** the cross section of the impactor (32) is smaller than the cross section of the retaining element (10).

27. The instrument set according to any one of claims 24 to 26, **characterized in that** the impactor (32) has a quadrangular cross section.

28. The instrument set according to any one of claims 24 to 27, **characterized in that** the impactor (32) comprises at least one graduation (62, 64, 66) at a distal portion.

29. The instrument set according to any one of claims 24 to 28, **characterized in that** the impactor (32) has a fork-shaped configuration.

30. The instrument set according to any one of claims 1 to 29, **characterized in that** it also comprises a ram (40) for positioning a transverse pin (28) in a bone.

## Revendications

1. Instrument servant à fixer un implant (26) de type cordon dans un os, comprenant un élément de maintien (10) pouvant être inséré dans l'os ainsi qu'un boulon transversal (28), sachant que l'élément de maintien (10) présente un évidement (14) servant à recevoir le boulon transversal (28) ainsi que des moyens pour relier ledit élément de maintien de manière amovible à un applicateur (34) et que l'élément de maintien sert en outre à la fixation de l'implant (26), sachant que le boulon transversal (28) ancre dans l'os l'élément de maintien (10) et sachant que l'élément de maintien (10) est constitué d'un matériau biodégradable, **caractérisé en ce que** l'élément de maintien (10) présente un guidage servant à recevoir l'implant (26), lequel guidage est réalisé de telle manière que l'implant (26) peut être guidé tout autour du boulon transversal (28) à la manière d'une boucle côté distal par l'élément de maintien (10).

2. Instrument selon la revendication 1, **caractérisé en ce que** le guidage est réalisé sous la forme d'un guidage ouvert.

3. Instrument selon la revendication 1, **caractérisé en ce que** le guidage est réalisé sous la forme d'un guidage fermé.

4. Instrument selon l'une quelconque des revendications 1 à 3, **caractérisé en ce qu'**un axe médian d'un boulon transversal (28) introduit dans l'évidement (14) se trouve de manière perpendiculaire sur un plan de boucle (24) défini par le guidage.

5. Instrument selon l'une quelconque des revendications 1 à 4, **caractérisé en ce que** l'élément de maintien (10) est réalisé de manière à présenter une forme de U.

6. Instrument selon l'une quelconque des revendications 1 à 5, **caractérisé en ce que** le guidage entoure l'évidement (14) sur au moins 180°.

7. Instrument selon l'une quelconque des revendications 1 à 6, **caractérisé en ce qu'**une extrémité proximale (16) de l'élément de maintien (10) présente une section transversale rectangulaire.

8. Instrument selon l'une quelconque des revendications 1 à 7, **caractérisé en ce que** l'élément de maintien (10) se rétrécit dans la direction distale de manière perpendiculaire par rapport au plan de boucle (24).

9. Instrument selon l'une quelconque des revendications 1 à 8, **caractérisé en ce que** l'évidement (14) s'élargit en direction du boulon transversal (28) de manière à présenter une forme d'entonnoir.

10. Instrument selon l'une quelconque des revendications 1 à 9, **caractérisé en ce que** les moyens sont réalisés sous la forme d'un mécanisme d'enclenchement.

11. Instrument selon l'une quelconque des revendications 1 à 10, **caractérisé en ce qu'**il présente en outre un applicateur (34) servant à introduire l'élément de maintien (10) dans un os.

12. Instrument selon la revendication 11, **caractérisé en ce que** l'applicateur (34) présente au moins un premier bras (70, 98) servant à introduire un élément de maintien (10) dans un os et au moins un deuxième bras (72) servant à introduire un boulon transversal (28) dans un os.

13. Instrument selon la revendication 12, **caractérisé en ce que** l'applicateur (34) présente deux premiers bras (70, 98) servant à introduire des éléments de maintien (10).

14. Instrument selon la revendication 13, **caractérisé en ce que** les deux premiers bras (70, 98) sont disposés de telle manière que les évidements (14) des éléments de maintien (10) placés au niveau des premiers bras (70, 98) sont alignés.

15. Instrument selon la revendication 14, **caractérisé en ce que** les deux premiers bras (70, 98) peuvent être coulissés les uns par rapport aux autres le long d'une ligne d'alignement des évidements (14) des éléments de maintien (10) placés au niveau des premiers bras (70, 98).

16. Instrument selon l'une quelconque des revendications 12 à 15, **caractérisé en ce que** le deuxième bras (72) au moins au nombre de un présente une douille de perçage (94).

17. Instrument selon l'une quelconque des revendications 12 à 16, **caractérisé en ce que** le premier bras au moins au nombre de un présente des moyens (70, 98) afin de relier ledit bras de manière amovible à un élément de maintien (10).

18. Instrument selon la revendication 17, **caractérisé en ce que** les moyens sont réalisés sous la forme d'un mécanisme d'enclenchement.

19. Instrument selon l'une quelconque des revendications 1 à 18, **caractérisé en ce qu'**il présente en outre un foret (38) servant à percer un canal pour un boulon transversal (28).

20. Instrument selon la revendication 19, **caractérisé en ce qu'**il présente en outre une fraise (36) servant à fraiser un canal pour un boulon transversal (28).

21. Instrument selon la revendication 20, **caractérisé en ce que** la fraise est réalisée sous la forme d'une fraise creuse (36).

22. Instrument selon la revendication 21, **caractérisé en ce que** la fraise (36) peut être couplée au foret (38).

23. Instrument selon la revendication 22, **caractérisé en ce que** le foret (38) dépasse de la fraise (36) à l'état couplé.

24. Instrument selon l'une quelconque des revendications 1 à 23, **caractérisé en ce qu'**il présente en outre un dispositif enfonceur (32) servant à enfoncer un canal pour un élément de maintien (10).

25. Instrument selon la revendication 24, **caractérisé en ce que** le dispositif enfonceur (32) présente une section transversale ayant une forme qui correspond à une section transversale d'un élément de maintien (10).

26. Instrument selon la revendication 25, **caractérisé en ce que** la section transversale du dispositif enfonceur (32) est plus petite que la section transversale de l'élément de maintien (10).

27. Instrument selon l'une quelconque des revendications 24 à 26, **caractérisé en ce que** le dispositif enfonceur (32) présente une section transversale rectangulaire.

28. Instrument selon l'une quelconque des revendications 24 à 27, **caractérisé en ce que** le dispositif enfonceur (32) présente au niveau de sa section distale au moins une graduation (62, 64, 66).

29. Instrument selon l'une quelconque des revendications 24 à 28, **caractérisé en ce que** le dispositif enfonceur (32) est réalisé de manière à présenter une forme de fourche.

30. Instrument selon l'une quelconque des revendications 1 à 29, **caractérisé en ce que** ledit instrument présente un outre un poussoir (40) servant à positionner un boulon transversal (28) dans un os.
